(19) Europäisches Patentamt

European Patent Office

Office européen des brevets

(11) Veröffentlichungsnummer: **0 679 412 A1**

## (12) EUROPÄISCHE PATENTANMELDUNG

(21) Anmeldenummer: **95106382.5**

(22) Anmeldetag: **27.04.95**

(51) Int. Cl.⁶: **A62D 3/00**

Der Mikroorganismus ist bei DSM 8962 unter der Nummer SK 37 hinterlegt worden.

(30) Priorität: **29.04.94 DE 4415127**

(43) Veröffentlichungstag der Anmeldung:
**02.11.95 Patentblatt 95/44**

(84) Benannte Vertragsstaaten:
**AT BE CH DE DK ES FR GB GR IE IT LI LU NL PT SE**

(71) Anmelder: **BOEHRINGER MANNHEIM GMBH**
**Sandhofer Strasse 112-132**
**D-68305 Mannheim-Waldhof (DE)**

(72) Erfinder: **Batz, Hans-Georg, Dr.**

**Traubinger Strasse 63**
**D-82327 Tutzing (DE)**
Erfinder: **Sluka, Peter, Dr.**
**St. Anna-Weg 17**
**D-82362 Weilheim (DE)**
Erfinder: **Jendrossek, Dieter, Dr.rer.nat.**
**Im Bache 22**
**D-37120 Bovenden (DE)**
Erfinder: **Steinbüchel, Alexander, Dr.**
**Rönnenthal 27**
**D-48341 Altenberge (DE)**

(74) Vertreter: **Weiss, Wolfgang, Dr. et al**
**Postfach 860820**
**D-81635 München (DE)**

(54) **Abbaubare Polymere.**

㊸ Die Erfindung betrifft ein neues Verfahren zur Beseitigung von durch biologische und potentiell infektiöse Substanzen kontaminierten Abfällen, deren feste Komponenten im wesentlichen aus Kunststoffen bestehen, ausgewählt aus der Gruppe bestehend aus Polyestern, Polylactiden, Polylactonen, Polycarbonaten und Gemischen davon, welches dadurch gekennzeichnet ist, daß man die Abfälle einer Behandlung unterzieht, die eine Inkubation in einem wäßrigen Medium in Gegenwart von Mikroorganismen oder/und ein Erhitzen in einem wäßrigen alkalischen Medium für eine ausreichende Zeitdauer umfaßt, um eine mindestens teilweise Auflösung der festen Kunststoffe zu bewirken.

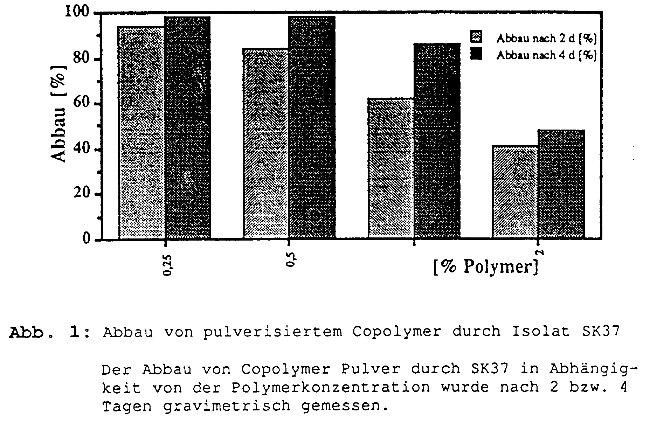

Abb. 1: Abbau von pulverisiertem Copolymer durch Isolat SK37

Der Abbau von Copolymer Pulver durch SK37 in Abhängigkeit von der Polymerkonzentration wurde nach 2 bzw. 4 Tagen gravimetrisch gemessen.

EP 0 679 412 A1

Die vorliegende Erfindung betrifft ein Verfahren zur Beseitigung von durch biologische und potentiell infektiöse Substanzen kontaminierten Abfällen, deren feste Komponenten im wesentlichen aus Kunststoffen bestehen, ausgewählt aus der Gruppe, bestehend aus Polyestern, Polylactiden, Polylactonen und Polycarbonaten.

Die Verwendung von Kunststoffen als Labormaterialien ist weitverbreitet. Kunststoffe können auf einfache Weise, z.B. durch Gießen, Pressen, Blas- und Warmverformen, Walzen und Extrudieren zu Formteilen gefertigt werden, die als Labormaterialien Anwendung finden, z.B. Pipettenspitzen, Reaktionsgefäße, Küvetten, Mikrotiterplatten und dgl..

Diese Kunststoffteile kommen in Labors oder Produktionsanlagen, in denen auf klinischem, molekularbiologischem, mikrobiologischem oder molekulargenetischem Gebiet gearbeitet wird, mit biologischen und potentiell infektiösen Substanzen in Kontakt. Beispiele solcher Substanzen sind Mikroorganismen, insbesonsdere genetisch manipulierte Mikroorganismen, wie etwa Bakterien, Pilze, Viren, Protozoen und dgl. sowie Pflanzenzellkulturen, Tierzellkulturen einschließlich menschlichen Zellkulturen, oder Körperflüssigkeiten, wie etwa Blut oder genetisches Material aus derartigen Organismen und Zellen. Die mit solchen Substanzen in Kontakt gekommenen Labormaterialien müssen als Sondermüll unter erheblichem Aufwand durch Verbrennen entsorgt werden. Da Labormaterialien aus Kunststoff in großem Umfang eingesetzt werden, entstehen auf diese Weise große Abfallmengen, zu deren Beseitigung ein erheblicher Kostenaufwand erforderlich ist. Zur Verringerung dieses Kostenaufwands besteht ein großes Bedürfnis.

Biologisch abbaubare Kunststoffe sind bekannt. Ein Übersichtsartikel von Buchner et al. (Erdöl, Erdgas, Kohle 106 (6), 259-262 (1990)) beschreibt die Herstellung, den Einsatz und die Eigenschaften von biologisch abbaubaren Polymeren, insbesondere von Polyhydroxyfettsäuren, wie Polyhydroxybuttersäure und Polyhydroxyvaleriansäure. Es wird beschrieben, daß sich Folien aus Polyhydroxybuttersäure im Boden nach 50 Tagen bis zu 40 % und nach 80 Tagen bis zu 70 % abbauen können.

Ein Artikel von Brandl und Püchner (NATO ASI SER., Ser. E, Vol. 186, 421-422 (1990)) beschreibt das Abbauverhalten von Shampooflaschen aus Polyhydroxyfettsäuren, insbesondere Polyhydroxybuttersäuren in Wasser unter natürlichen Bedingungen. Die hochgerechnete Lebensdauer dieser Flaschen in Wasser beträgt 5 bis 10 Jahre bis zum vollständigen Abbau.

Kumagai und Doi (Polymer Degradation and Stability 35, 87-93 (1992)) beschreiben Experimente zum enzymatischen Abbau von Mischungen aus Polyhydroxybuttersäure und Polyethylenoxid. Bei Mischpolymeren erfolgt ein kompletter enzymatischer Abbau, wobei die Abbaugeschwindigkeit stark vom Polyethylenoxidgehalt abhängig ist. Die hohe Abbaugeschwindigkeit ist auf die Hydrolyse der Polyethylenoxidkomponente in wäßriger Lösung bei 37°C zurückzuführen.

Coffin und McGinity (Pharmaceutical Research, Vol. 9, 200-205 (1992)) beschreiben eine Untersuchung zur chemischen Stabilität von kolloidalen Partikeln aus p-Lactid und p-Caprolacton in wäßrigen Dispersionen. Bei 37°C wird ein rascher Abbau der Polymere gefunden. Bei einer Verringerung des pH-Werts wird eine starke Abnahme der Stabilität gefunden.

Mukai et al. (Biotechnology Letters 15 (6), 601-604 (1993)) beschreiben Untersuchungen zum Abbauverhalten von Filmen aus fünf verschiedenen Polyhydroxyfettsäuren im wäßrigen Medium in Gegenwart von Enzymen (Lipasen und PHA-Depolymerasen). Der Abbau erfolgt innerhalb eines Zeitraums von 5 Stunden bei 37°C und hohen Enzymkonzentrationen nur partiell.

Die Produktbroschüre Biopol® der Firma ZENECA (1993) beschreibt die zur Zeit auf dem Markt erhältlichen Typen von biologisch abbaubaren Polyhydroxyfettsäuren. Es finden sich ausführliche Angaben zu den physikalischen Eigenschaften und Verarbeitungshinweise. Es finden sich auch Angaben über die Abbaubarkeit unter verschiedenen Bedingungen. Tests im Belebtschlamm zeigen unter aeroben Bedingungen einen Abbau von maximal 85 % nach 40 Tagen und unter anaeroben Bedingungen einen Abbau von maximal 78 % nach 30 Tagen. Ein Abbau in mikrobiell aktivem Abwasser unter aeroben Bedingungen ergab nach 25 Wochen einen Gewichtsverlust von 50 %.

In keiner der oben genannten Literaturstellen findet sich der Vorschlag, als Labormaterialien, die mit biologischen und potentiell infektiösen Substanzen in Kontakt kommen können, biologisch abbaubare Kunststoffe zu verwenden. Weiterhin sind die aus dem Stand der Technik bekannten Abbaugeschwindigkeiten entweder zu gering, um von praktischem Nutzen bei der Beseitigung von Kunststoffabfällen zu sein, oder der Abbau findet unter solchen Bedingungen statt, die für eine großtechnische Praxis völlig ungeeignet sind.

Die der vorliegenden Erfindung zugrundeliegende Aufgabe bestand somit darin, ein schnelles und umweltschonendes Verfahren zur Beseitigung von Kunststoffabfällen aus Labors, Industrieanlagen oder Kliniken bereitzustellen, bei dem die Nachteile von Verfahren des Standes der Technik mindestens teilweise überwunden werden.

Diese Aufgabe wird gelöst durch ein Verfahren zur Beseitigung von durch biologische und potentiell infektiöse Substanzen kontaminierten Abfällen, deren feste Komponenten im wesentlichen aus Kunststoffen bestehen, ausgewählt aus der Gruppe, bestehend aus Polyestern, Polylactiden, Polylactonen, Polycarbonaten und Gemischen davon, wobei das Verfahren dadurch gekennzeichnet ist, daß man die Abfälle einer Behandlung unterzieht, die eine Inkubation in einem wäßrigen Medium in Gegenwart von Mikroorganismen oder/und ein Erhitzen in einem wäßrigen alkalischen Medium für eine ausreichende Zeitdauer umfaßt, um eine mindestens teilweise Auflösung der festen Kunststoffe zu bewirken.

Die im erfindungsgemäßen Verfahren eingesetzten Abfälle sind feste Kunststoffabfälle, in denen gegebenenfalls noch Reste der biologischen Substanzen vorhanden sein können, mit denen sie während ihres Einsatzes in Kontakt gekommen sind. Beispiele für solche Substanzen sind Mikroorganismen, Zellen, Körperflüssigkeiten, insbesondere Blutprodukte, Zellbestandteile, wie etwa Nucleinsäuren und Proteine und dgl.. Weiterhin können die Kunststoffabfälle noch Kulturmedienrückstände sowie Rückstände von Laborreagenzien enthalten.

Vorzugsweise sind die eingesetzten Kunststoffe Polyester, besonders bevorzugt aliphatische Polyester, insbesondere Polyhydroxyfettsäure-(PHF)-Polymere oder PHF-Copolymere, die einen PHF-Anteil von vorzugsweise mindestens 30 Gew.-%, vorzugsweise mindestens 50 Gew.-% und besonders bevorzugt mindestens 70 Gew.-% enthalten. Spezifische Beispiele von Copolymeren sind Polyhydroxyfettsäure-Polylacton-Copolymere.

Unter Polyhydroxyfettsäure-Polymeren sind gemäß vorliegender Erfindung Polymere zu verstehen, die aus einem oder mehreren $C_3$-$C_{12}$-Hydroxycarbonsäure-Monomeren aufgebaut sind. Bevorzugt sind $C_3$-$C_6$-Hydroxycarbonsäuren, besonders bevorzugt $C_4$- und $C_5$-Hydroxycarbonsäuren, insbesondere $\beta$-Hydroxybuttersäure und $\beta$-Hydroxyvaleriansäure.

Die Abfälle werden einer Behandlung in einem wäßrigen Medium für eine ausreichende Dauer unterzogen, um eine mindestens teilweise Auflösung der festen Kunststoffe zu bewirken. Nach diesem Behandlungsschritt kann, sofern erforderlich, noch eine Sterilisation des wäßrigen Mediums auf übliche Weise, z.B. durch Erhitzen und/oder Bestrahlung erfolgen. Diese Maßnahme kann insbesondere bei einem Abbau der Abfälle durch Mikroorganismen noch erforderlich sein.

Durch das erfindungsgemäße Verfahren wird eine mindestens teilweise Auflösung der festen Kunststoffe bewirkt. Vorzugsweise werden die Kunststoffe zu mindestens 50 %, besonders bevorzugt zu mindestens 80 % und am meisten bevorzugt vollständig aufgelöst. Bei vollständiger Auflösung der Kunststoffe können inerte Partikel aus anorganischem Material, die zur Nuklierung oder als Pigmente verwendet werden, z.B. BN, $TiO_2$, $SiO_2$ und dgl. zurückbleiben.

Die zur gewünschten Auflösung der festen Kunststoffe ausreichende Zeitdauer bestimmt sich nach der Art der Behandlung. In einer ersten Ausführungsform umfaßt die Behandlung eine Inkubation der Kunststoffabfälle in einem wäßrigen Medium in Gegenwart von Mikroorganismen. In dieser Ausführungsform werden als Kunststoffe vorzugsweise Polyhydroxybuttersäure-Copolymere eingesetzt, die günstigerweise einen Gewichtsanteil von 10 bis 99,5 %, insbesondere 20 bis 98 % Polyhydroxybuttersäure enthalten. Besonders bevorzugt sind Copolymere aus Polyhydroxybuttersäure und mindestens einem anderen Polyhydroxyfettsäure-Monomer. Am meisten bevorzugt sind Kunststoffe, deren organische Polymerkomponenten im wesentlichen, d.h. > 95 % aus Hydroxybuttersäure- und Hydroxyvaleriansäure-Monomeren aufgebaut sind, d.h. Polyhydroxybuttersäure-Polyhydroxyvaleriansäure-Copolymere (PHB-co-PHV). Die im erfindungsgemäßen Verfahren eingesetzten PHB-co-PHV-Polymere haben vorzugsweise einen Gewichtsanteil von 1 bis 50 %, besonders bevorzugt von 5 bis 30 % Hydroxyvaleriansäure.

Bei der mikrobiellen Behandlung werden die Kunststoff-Abfälle dem wäßrigen Medium in einem solchen Gewichtsanteil zugesetzt, der einerseits groß genug ist, um den Abbau großer Kunststoffmengen zu ermöglichen, und andererseits gering genug ist, um einen effektiven Abbau innerhalb kurzer Zeit zu ermöglichen. Die Kunststoffe werden daher vorzugsweise in einem Gewichtsanteil von 0,01 bis 5 % des Gewichts des wäßrigen Mediums eingesetzt, um optimale Auflösungsgeschwindigkeiten zu erhalten. Besonders bevorzugt wird der Kunststoff in einem Gewichtsanteil von 0,1 bis 2,5 Gew.-% und am meisten bevorzugt 0,5 bis 1,5 % auf Basis des Gewichts des festen Mediums eingesetzt.

Überraschenderweise wurde jedoch festgestellt, daß auch sehr hohe Konzentrationen von Polymeren mit großer Geschwindigkeit abgebaut werden können. So konnte beispielsweise bei einem Gewichtsanteil von 5 % Polymer im Medium innerhalb von 11 Tagen ein Abbau von 57 % erzielt werden. Daher kann in einem optimierten Medium ohne weiteres auch ein Polymer-Anteil von bis zu 10 Gewichtsprozent verwendet werden.

Die mikrobielle Behandlung der Kunststoffabfälle findet bei der Temperatur statt, wo die günstigsten Wachstumsbedingungen für die jeweils eingesetzten Mikroorganismen herrschen. Diese Temperatur beträgt üblicherweise 10 bis 50 °C und insbesondere 20 bis 40 °C.

Die Kunststoffe können für das erfindungsgemäße Verfahren als Formteile, d.h. ohne vorherige Zerkleinerung, eingesetzt werden. Die Abbaugeschwindigkeit kann jedoch erhöht werden, wenn die Kunststoffe zuvor durch eine geeignete Zerkleinerungsvorrichtung in Granulate bzw. Pulver überführt werden. Natürlich können die eingesetzten Kunststoffmaterialien teilweise in zerkleinerter und unzerkleinerter Form eingesetzt werden.

Die zum Abbau der Polymere verwendeten Mikroorganismen können sowohl aerobe als auch anaerobe Mikroorganismen sein. Ein Beispiel für aerobe Mikroorganismen ist Belebtschlamm aus einer Kläranlage, und ein Beispiel für anaerobe Mikroorganismen ist Faulschlamm aus einer Kläranlage. Bevorzugt sind aerobe Mikroorganismen, insbesondere solche, die zur Verwertung von Polyhydroxyfettsäuren in der Lage sind. Derartige Mikroorganismen können nach den bei Jendrossek et al. (J. Environ. Polym. Degrad. 1 (1993) 53-63) und den darin zitierten Arbeiten beschriebenen Methoden isoliert werden.

Die optimalen Behandlungsbedingungen sind von den jeweils verwendeten Mikroorganismen abhängig. Bei aeroben Mikroorganismen ist die Zufuhr von Luft in das wäßrige Medium bevorzugt, während es bei anaeroben Kulturen die Zersetzung der Kunststoffe vorzugsweise ohne von außen zugeführte Luft erfolgt. Die Kultivierung kann in mineralischer Nährlösung, gegebenenfalls unter Zusatz von geringen Mengen (0,05 bis 0,5 %) Bakterien- oder Hefeextrakt erfolgen. Die bevorzugte Inkubationstemperatur für aerobe Kulturen beträgt 25 bis 35 °C, insbesondere 30 °C und für anaerobe Kulturen 30 bis 40 °C, insbesondere 33 bis 37 °C. Die Inkubation findet vorzugsweise in einer mineralischen Nährlösung statt, die einen pH-Wert von vorzugsweise 6 bis 9,5 aufweist. Eine derartige Nährlösung kann z.B. als Komponenten Na, K, Mg, Ca, Fe, Spurenelemente (Zn, Mn, B, Co, Cu, Ni, Mo etc.), Ammonium oder/und Phosphat enthalten.

Besonders bevorzugt weist die mineralische Nährlösung einen alkalischen pH-Wert, insbesondere einen pH-Wert von 8 - 9, z.B. 8,5 auf. Der pH-Wert der Nährlösung kann durch Zugabe einer geeigneten Base, z.B. Tris, erhöht werden.

Weiterhin wurde festgestellt, daß die Abbauleistung durch Optimierung der Ammonium-, Eisen-, oder/und Calciumionenkonzentration deutlich verbessert werden kann.

Die Ammoniumionenkonzentration beträgt vorzugsweise 0,10 - 0,30 %, besonders bevorzugt 0,15 - 0,25 % (berechnet als $NH_4$ Cl), bezogen auf das Gewicht des Mediums. Die Calciumionenkonzentration beträgt vorzugsweise mindestens 0,001 %, besonders bevorzugt 0,001 - 0,01 % (berechnet als $CaCl_2 \times 2\ H_2O$) bezogen auf das Gewicht des Mediums. Die Eisenionenkonzentration beträgt vorzugsweise mindestens 0,0005 %, besonders bevorzugt 0,0005 - 0,005 % (berechnet als $FeNH_4$-Citrat) bezogen auf das Gewicht des Mediums.

Ein bevorzugter Mikroorganismus ist das aerobe Isolat SK 404, das als Pseudomonas lemoignei bereits von C.J. Lusty und M. Doudoroff (Proc. Natl. Acad. Sci. 56, 3 (1966), 960-965) beschrieben wurde. Ein weiterer bevorzugter Mikroorganismus ist das aerobe Isolat SK 37, das bei der DSM - Deutsche Sammlung von Mikroorganismen und Zellkulturen GmbH, Mascheroder Weg 1b, D-38124 Braunschweig am 09. Februar 1994 unter der Nummer DSM 8962 hinterlegt wurde, sowie äquivalente Mikroorganismen, d.h. Mikroorganismen mit äquivalenter Abbauleistung. Besonders bevorzugt ist das Isolat SK 37 oder ein äquivalenter Mikroorganismus.

Die vorliegende Erfindung betrifft somit auch die Verwendung des Mikroorganismus SK 37 oder von Mikroorganismen mit äquivalenten Abbauleistungen zur mindestens teilweisen Verflüssigung von biologisch abbaubaren Kunststoffen in einem wäßrigen Medium.

Bei dem erfindungsgemäßen Verfahren ist eine Verringerung des Gewichts der festen Kunststoffe um mindestens 50 % innerhalb einer Behandlungsdauer von maximal 30 Tagen, vorzugsweise maximal 20 Tagen und besonders bevorzugt maximal 10 Tagen möglich. Besonders bevorzugt verringert sich das Gewicht der festen Kunststoffe innerhalb dieses Zeitraums um mindestens 80 % und am meisten bevorzugt werden die festen Kunststoffe innerhalb dieses Zeitraums vollständig (d.h. die organischen Komponenten des Kunststoffes) aufgelöst.

Der erfindungsgemäße mikrobielle Abbau von Kunststoffen kann einerseits chargenweise durchgeführt werden, d.h. eine bestimmte Kunststoffmenge wird in einem wäßrigen Medium für eine bestimmte Zeitdauer in Gegenwart von Mikroorganismen inkubiert. Nach Beendigung der Inkubation kann das flüssige Medium, gegebenenfalls nach Sterilisierung, beseitigt werden. Andererseits ist auch eine kontinuierliche Verfahrensführung möglich, d.h. dem Inkubationsbehälter wird immer nur ein Teil des Mediums entnommen und dafür frisches Medium und frische Kunststoffabfälle zugesetzt. Bei einer derartigen, für großtechnische Anwendungen bevorzugten Verfahrensführung wird günstigerweise eine pH-Kontrolle des Mediums durchgeführt, da der pH-Wert des Mediums aufgrund der Zersetzung der Polymere stark absinken kann, so daß keine guten Wachstumsbedingungen mehr für die verwendeten Mikroorganismen herrschen. Vorzugsweise wird der pH-Wert daher durch Zugabe von alkalischen Substanzen, z.B. Alkalimetallhydroxide, Alkalimetallcarbonate, Ammoniumhydroxid und dgl. auf einen Bereich von 6 bis 8 eingestellt.

In einer weiteren Ausführungsform des erfindungsgemäß Verfahrens umfaßt die Behandlung ein Erhitzen der Kunststoffabfälle in einem wäßrigen alkalischen Medium. Bei dieser Ausführungsform der vorliegenden Erfindung verwendet man vorzugsweise Kunststoffe, in denen feste anorganische Partikel dispergiert sind. Der Gewichtsanteil der anorganischen Partikel beträgt vorzugsweise 0,1 bis 15 % und besonders bevorzugt 1 bis 10 % auf Basis des Kunststoffgesamtgewichts. Die anorganischen Partikel haben eine Größe im Bereich von 1 nm bis 100 $\mu$m, vorzugsweise im Bereich von 10 nm bis 10 $\mu$m und besonders bevorzugt von 100 nm bis 1 $\mu$m.

Auch in dieser Ausführungsform der Erfindung werden vorzugsweise Polyester, besonders bevorzugt aliphatische Polyester und insbesondere Polyhydroxyfettsäure-Polymere oder -Copolymere verwendet, die einen PHF-Anteil von mindestens 30 Gew.-%, vorzugsweise von mindestens 50 Gew.-% und besonders bevorzugt von mindestens 70 Gew.-% enthalten.

Die anorganischen Partikel können beispielsweise aus inerten anorganischen Materialien wie BN, $TiO_2$, $SiO_2$ und dgl. bestehen, die während der Herstellung der Kunststoffe als Nuklierungsmittel oder/und als Pigmentierungsmittel verwendet werden können. Spezifische Beispiele für Polymere, die in dieser Ausführungsform der Erfindung eingesetzt werden können, sind die kommerziell erhältlichen "Biopol"-Polymere, z.B. die Typen D300G, D310G, D400G, D410G, D600G und D610G, die nach Angaben des Herstellers Nuklierungsmittel zur Beschleunigung der Verarbeitungszyklen enthalten.

Die alkalische Behandlung der Polymere umfaßt ein Erhitzen auf vorzugsweise mindestens 90°C, besonders bevorzugt bis zum Siedepunkt des wäßrigen Mediums (im allgemeinen 100°C).

Das wäßrige alkalische Medium weist vorzugsweise einen pH-Wert von mindestens 13, besonders bevorzugt von mindestens 14 auf. Ein derartiger pH-Wert kann beispielsweise durch Zusatz von Alkalimetallhydroxid in einer Konzentration von 0,1 mol/l, vorzugsweise mindestens 0,5 mol/l bis vorzugsweise 10 mol/l zum wäßrigen Medium erreicht werden. Für praktische Anwendungen hat sich eine Konzentration von Alkalimetallhydroxid von 1 mol/l als ausreichend erwiesen, um Kunststoffe innerhalb kurzer Zeit vollständig aufzulösen.

Bei dieser Ausführungsform der vorliegenden Erfindung wird der Kunststoff im wäßrigen alkalischen Medium in einem Gewichtsanteil von vorzugsweise 1 bis 15 %, besonders bevorzugt von 2 bis 8 % auf Basis des Gesamtvolumens des Mediums eingesetzt.

Überraschenderweise wurde festgestellt, daß sich die organische Komponente des Polymers bei Auskochen mit 1 mol/l NaOH bereits in 1 Stunde auflösen kann. Die Dauer des Erhitzens richtet sich im allgemeinen nach dem Alkaligehalt des Mediums und der Menge an verwendetem Polymer, im allgemeinen beträgt die Dauer des Erhitzens 30 Minuten bis 24 Stunden, vorzugsweise 30 Minuten bis 2 Stunden.

In noch einer weiteren Ausführungsform der vorliegenden Erfindung kann die Behandlung der Kunststoffabfälle die Kombination einer Inkubation in Gegenwart von Mikroorganismen und eines Erhitzens unter alkalischen Bedingungen umfassen. Bei dieser Ausführungsform erfolgt vorzugsweise zuerst die Inkubation in Gegenwart von Mikroorganismen und nach dieser Verfahrensstufe gegebenenfalls zurückbleibende feste Polymerrückstände können anschließend beim Erhitzen in alkalischem Medium aufgelöst werden. Diese Kombination der Verfahrensstufen hat den Vorteil, daß zumindest der größte Teil der festen Kunststoffabfälle mikrobiell, d.h. ohne Einsatz aggressiver Chemikalien und ohne Energiezufuhr, aufgelöst werden kann und gegebenenfalls verbleibende geringe Rückstände vollständig innerhalb kürzester Zeitdauer durch die alkalische Behandlung beseitigt werden können. Auf diese Weise können auch große Mengen an Kunststoffabfällen innerhalb vertretbarer Zeitspannen kostengünstig und umweltfreundlich entsorgt werden.

Noch ein weiterer Gegenstand der vorliegenden Erfindung ist die Verwendung von Polyhydroxybuttersäure-Copolymeren zur Herstellung von Kunststoff-Labormaterialien, die innerhalb eines Zeitraums von mindestens 20 Tagen mikrobiell vollständig abbaubar sind.

Weiterhin betrifft die Erfindung die Verwendung von Polyhydroxyfettsäure-Polymeren, die dispergierte anorganische Partikel enthalten, zur Herstellung von Kunststoff-Labormaterialien, die innerhalb eines Zeitraums von 24 Stunden durch Erhitzen unter alkalischen Bedingungen vollständig abbaubar sind.

Diese Kunststoff-Labormaterialien sind vorzugsweise Formteile, ausgewählt aus der Gruppe, umfassend Pipettenspitzen, Reaktionsgefäße wie etwa Mikroreaktionsgefäße (z.B. mit einem Reaktionsvolumen von 100 $\mu$l bis 5 ml), Küvetten und Mikrotiterplatten.

Weiterhin wird die vorliegende Verbindung durch die folgenden Beispiele erläutert.

Beispiel 1

Chemischer Abbau von Polyhydroxyfettsäure-Polymeren

Eine Probe von ca. 1 g Biopol Typ 300G (Copolymer-Granulat mit geringem Hydroxyvaleriansäure-Anteil) der Firma ZENECA, Ltd. wurde 1 Stunde lang in 20 ml 1 mol/l NaOH unter Rückfluß ausgekocht. Das Polymer löst sich während dieser Zeit vollständig auf. Als feiner Niederschlag bleibt ca. 50 mg des Nuklierungsmittels zurück. Bei Verwendung von 0,1 mol/l NaOH zersetzt sich das Material unter gleichen Bedingungen innerhalb eines Zeitraums von 24 Stunden. Auch Copolymere aus Biopol und Polylactid zersetzen sich bei Erhitzen in Gegenwart von Natronlauge, wobei aber im beobachteten Zeitraum lediglich das Biopol-Material vollständig abgebaut wird.

Beispiel 2

Mikrobiologischer Abbau von Polyhydroxyfettsäure-Polymeren

**1. Polymermaterialien**

Es wurden folgende Polymere verwendet:

(i) der Homopolyester aus 3-Hydroxybuttersäure (PHB),

(ii) der Homopolyester aus 3-Hydroxyvaleriansäure (PHV) und

(iii) ein Copolyester aus 3-Hydroxybuttersäure (82 mol-%) und 3-Hydroxyvaleriansäure (18 mol-%), PHB/HV.

Die Polymere (i) und (iii) wurden von der Firma ICI bezogen. Das PHV-Homopolymer kann aus dem Bakterium Chromobacterium violaceum ausgehend von Valeriansäure als Kohlenstoffquelle hergestellt werden (siehe Steinbüchel et al., Appl. Microbiol. Biotechnol. 39 (1993) 443-493).

**2. Organismen**

Es wurden folgende Misch- und Reinkulturen verwendet:

Belebtschlamm aus der Göttinger Kläranlage (aerob)

Faulschlamm aus der Göttinger Kläranlage (anaerob)

Isolat SK 37 (DSM 8962) (aerob)

Isolat SK 404 (Pseudomonas lemoignei; C.J. Lusty und M. Doudoroff, Proc. Natl. Acad. Sci. 56, 3 (1966), 960-965) (aerob)

**3. Medium**

| H16-Medium: | | |
|---|---|---|
| a) | $Na_2HPO_4 \cdot 12\ H_2O$ | 0,90 % |
| | $KH_2PO_4$ | 0,15 % |
| | $NH_4Cl$ | 0,10 % |
| | $MgSO_4 \cdot 7\ H_2O$ | 0,02 % |
| | Spurenelement-Lösung (100-fach) | 0,1 ml/l |
| b) | Eisenlösung | 10,0 ml/l |
| a) und b) getrennt autoklaviert pH = 6,9 | | |

| Eisenlösung | |
|---|---|
| $FeNH_4$ Citrat | 0,05 % |
| $CaCl_2 \cdot 2H_2O$ | 0,10 % |

| Spurenelement-Lösung | |
|---|---|
| Titriplex III | 0,050 % |
| $FeSO_4 \cdot 7 H_2O$ | 0,020 % |
| $ZnSO_4 \cdot 7 H_2O$ | 0,0010 % |
| $MnCl_2 \cdot 4 H_2O$ | 0,0003 % |
| $H_3BO_3$ | 0,0030 % |
| $CoCl_2 \cdot 6 H_2O$ | 0,0020 % |
| $CuCl_2 \cdot 2 H_2O$ | 0,0001 % |
| $NiCl_2 \cdot 6 H_2O$ | 0,0002 % |
| $Na_2MoO_4 \cdot 2 H_2O$ | 0,0003 % |

### 4. Kulturbedingungen

Alle aeroben Kulturen wurden in 10 bis 25 ml mineralischer Nährlösung pH 6,9 (H-16 Medium) in 100 ml Erlenmeyerkolben bei 30°C unter Schütteln inkubiert. Anaerobe Kulturen mit Faulschlamm wurden in 10 ml mineralischer Nährlösung + 0,1 % Hefeextrakt in verschlossenen Röhrchen bei 37°C inkubiert. Als Inokulum wurden 0,1 Vol. Belebtschlamm und 0,3 Vol. Faulschlamm eingesetzt. Für Abbauversuche mit den Isolaten SK 37 und SK 404 wurden 0,2 Vol. einer 24 Stunden alten Vorkultur eingesetzt. Als Kohlenstoffquellen wurden die oben angegebenen Polymere verwendet.

### 5. Vorbehandlung der Polymere

Aus den jeweiligen Polymeren wurden durch Spritzguß Platten in der Größe von 15 x 15 cm x 0,1 cm und einem Gewicht von ca. 25 g hergestellt. Das Spritzgießen wurde bei Temperaturen von 178°C (PHB), 162°C (PHB/HV) und 113°C (PHV) durchgeführt. Für die Abbauversuche wurden Stücke aus diesen Platten verwendet.

Die Polymere wurden entweder in Form von Plättchen (ca. 10 mm x 5 mm x 1 mm, 70 bis 95 mg) oder als feinkörniges Pulver (ca. 60 bis 500 mg) eingesetzt. Die Plättchen wurden durch 5-minütige Inkubation in 70 % Ethanol teilentkeimt. Je nach Versuch wurden die Plättchen direkt eingesetzt oder für 15 Minuten in 0,1 N NaOH oder 0,1 N HCl bei Raumtemperatur oder im kochenden Wasserbad vorbehandelt. Zur Pulverisierung wurden die Polymerplatten mit einer Schere in ca. 3 x 3 cm große Stücke zerschnitten, auf ca. 20°C gekühlt und in einer ebenfalls im Gefrierschrank vorgekühlten elektrischen Kaffeemühle für ca. 3 Minuten zermahlen. Das Pulver wurde durch ein feinmaschiges Teesieb in eine grobkörnige und eine feinkörnige Fraktion getrennt. Um einheitliche Bedingungen zu gewährleisten, wurde nur die feinkörnige Fraktion verwendet.

### 6. Bestimmung des Polymerabbaus

Der Abbau der Polymere wurde gravimetrisch als Differenz zwischen Ein- und Auswaage erfaßt. Dazu wurden die Polymerplättchen nach Versuchsende durch kurzes Abspülen mit Wasser von anhaftenden Organismen befreit und im Vakuum über Trockenmittel getrocknet. Bei Versuchen mit Polymerpulver wurde die gesamte Kultur über ein 12 $\mu$m-Filter geleitet und die zurückgehaltenen Partikel nach Trocknung gewogen. Um zu prüfen, ob alle Polymerpartikel vom Filter zurückgehalten werden, wurde in exemplarischen Parallelversuchen die Menge an verbliebenem Polymer nach Zentrifugation aller Partikel und Lösung des Polymeranteils in Chloroform und anschließender saurer Methanolyse gaschromatographisch bestimmt. Bei dieser Methode wird allerdings das von den Zellen intrazellulär gespeicherte Polymer zusätzlich erfaßt. Als Kontrollen wurde analoge Versuche mit steriler Nährlösung durchgeführt.

### 7. Ergebnisse

7.1 Anaerober Abbau von PHF-Plättchen durch Faulschlamm

Es wurde der Abbau von Plättchen aus PHB, PHV und dem Copolymer durch Faulschlamm unter den oben genannten Bedingungen untersucht. Nach 32 Tagen wurde ein maximaler Gewichtsverlust von 39 % (PHB), 1,1 % (PHV) und 47 % (Copolymer) ermittelt. Die Art der Vorbehandlung hatte keinen signifikanten Einfluß auf die Abbaugeschwindigkeit.

7.2 Aerober Abbau von PHF-Plättchen durch Belebtschlamm und Reinkulturen

Es wurde der Abbau von Plättchen aus PHB, PHV und dem Copolymer unter anaeroben Bedingungen durch Belebtschlammorganismen sowie durch die Isolate SK 37 und SK 404 untersucht. Beide Organismen waren aufgrund ihrer Fähigkeit, PHB als alleinige Kohlenstoff- und Energiequelle zu nutzen, isoliert worden (vgl. z.B. Jendrossek al., J. Environ. Polym. Degrad. 1 (1993), 53-63 und darin zitierte Arbeiten).

Es zeigte sich, daß PHB-Plättchen gut durch Belebtschlammorganismen abgebaut wurden (42 - 48 % innerhalb von 27 Tagen). Das Isolat SK 37 hydrolysiert PHB ebenfalls gut (35 - 44 % innerhalb von 27 Tagen). PHV wurde von SK 37 und SK 404 innerhalb von 27 Tagen bis maximal 21 % abgebaut. Dagegen erfolgte der Abbau der Copolymerplättchen am besten. Durch Belebtschlammorganismen wurde ein Abbau bis zu 73 Gew.-% innerhalb von 27 Tagen erreicht. Noch schneller erfolgte der Abbau durch die beiden Reinkulturen. Teilweise wurde ein völliger Abbau bereits nach 20 Tagen festgestellt. Eine Vorbehandlung mit heißer NaOH beschleunigte den Abbau geringfügig. In allen Kontrollen ohne Mikroorganismen wurde kein signifikanter Gewichtsverlust im gesamten Versuchszeitraum festgestellt. Die Ergebnisse für PHB/HV sind in Tabelle 1 dargestellt.

7.3 Aerober Abbau von pulverisierten PHF durch das Isolat SK 37

Es wurde der Abbau von pulverisiertem PHB, PHV und Copolyester durch SK 37 untersucht (Tabelle 2). Dabei wurden für 25 ml Medium jeweils ca. 62,5, 125 oder 250 mg Polymerpulver eingesetzt (entsprechend einer Konzentration von 0,25, 0,5 bzw. 1,0 % bezogen auf das Medium) und nach 4 bzw. 6 Tagen ausgewertet. Zusätzlich wurde eine Vorbehandlung des Polymers durch Inkubation in heißer 0,1 mol/l NaOH auf die Abbaugeschwindigkeit untersucht.

PHV wurde erneut am schlechtesten abgebaut (Maximum 13 %). PHB wurde deutlich besser abgebaut: Nach 6 Tagen waren bereits 41 bis 71 % hydrolysiert. Das Copolymer wurde wiederum am besten abgebaut. Bereits nach 4 Tagen waren 91 bis 98 % hydrolysiert. Bei den Ansätzen mit 1,0 % Polymer war der absolute Abbau am größten. Die Vorbehandlung hatte keinen signifikanten Einfluß auf die Abbaugeschwindigkeit. Die Ergebnisse für PHB und PHB/HV sind in Tabelle 2 dargestellt.

In Abbildung 1 ist das Ergebnis eines weiteren Abbauversuchs des Copolyesters durch SK 37 dargestellt. Bei Zugabe des Polyesters in Konzentrationen von 0,25 bis 2,0 % in das Medium wurde innerhalb von 2 bis 4 Tagen ein Abbau von 41 bis 99 % beobachtet. Die günstigsten Ergebnisse wurden bei einer Konzentration von etwa 1 Gew.-% Polymer erhalten. Hierbei wurden nach 2 Tagen 62 % und nach 4 Tagen 86 % Abbau erreicht. Höhere Polymerkonzentrationen (2 %) führten zu einer geringen Steigerung des absoluten und zu einer deutlichen Verminderung des prozentualen Abbaus und bewirkten eine pH-Wertabsenkung auf unter 5.

Tabelle 1: Aerober Abbau von PHB/HV-Plättchen

| Organismus | Vorbehandlung | Dauer [d] | Einwaage [mg] | Rückwaage [mg] | Abbau [%] | pH-Wert bei Ernte |
|---|---|---|---|---|---|---|
| -- | --- | 27 | 92,1 | 92,4 | 0 | 6,90 |
| -- | HCl, RT | 27 | 100,4 | 100,7 | 0 | 6,89 |
| -- | NaOH, RT | 27 | 94,7 | 94,9 | 0 | 6,89 |
| -- | NaOH, RT | 27 | 101,3 | 101,7 | 0 | 6,88 |
| -- | NaOH, 100° | 27 | 98,8 | 99,1 | 0 | 6,97 |
| SK 37 | --- | 8 | 82,8 | 57,7 | 30,3 | 7,19 |
| " | --- | 20 | 85,2 | <0,1 | 100,0 | 7,07 |
| " | --- | 27 | 84,8 | kein Rest | 100,0 | 7,06 |
| " | HCl, RT | 8 | 99,0 | 58,2 | 41,2 | 7,12 |
| " | HCl, RT | 20 | 85,7 | 31,9 | 62,8 | 7,14 |
| " | HCl, RT | 27 | 80,8 | 16,8 | 79,2 | 7,09 |
| " | HCl, 100° | 8 | 81,4 | 49,1 | 39,7 | 7,17 |
| " | HCl, 100° | 20 | 80,3 | kein Rest | 100,0 | 7,10 |
| " | HCl, 100° | 27 | 85,9 | kein Rest | 100,0 | 6,93 |
| " | NaOH, RT | 8 | 91,2 | 49,0 | 46,3 | 7,03 |
| " | NaOH, RT | 20 | 99,7 | kein Rest | 100,0 | 6,98 |
| " | NaOH, RT | 27 | 105,3 | kein Rest | 100,0 | 6,98 |
| " | NaOH, 100° | 8 | 79,9 | 26,6 | 66,7 | 7,08 |

| Organismus | Vorbehandlung | Dauer [d] | Einwaage [mg] | Rückwaage [mg] | Abbau [%] | pH-Wert bei Ernte |
|---|---|---|---|---|---|---|
| SK 37 | NaOH, 100° | 20 | 83,3 | kein Rest | 100,0 | 7,12 |
| " | NaOH, 100° | 27 | 83,2 | kein Rest | 100,0 | 7,06 |
| SK 404 | --- | 8 | 96,2 | 81,3 | 15,5 | 8,03 |
| " | --- | 20 | 101,0 | 9,8 | 90,3 | 7,83 |
| " | --- | 27 | 82,6 | 6,7 | 91,9 | 7,56 |
| " | HCl, RT | 8 | 86,0 | 69,5 | 19,2 | 9,00 |
| " | HCl, RT | 20 | 83,8 | 52,6 | 37,2 | 7,70 |
| " | HCl, RT | 27 | 84,6 | 0,9 | 98,9 | 7,55 |
| " | HCl, 100° | 8 | 81,1 | 55,0 | 32,2 | 7,86 |
| " | HCl, 100° | 20 | 102,6 | 29,7 | 71,1 | 7,75 |
| " | HCl, 100° | 27 | 90,0 | 10,7 | 88,1 | 7,44 |
| " | NaOH, RT | 8 | 104,6 | 73,0 | 30,2 | 7,72 |
| " | NaOH, RT | 20 | 87,5 | 6,1 | 93,0 | 7,43 |
| " | NaOH, RT | 27 | 108,1 | 24,9 | 77,0 | 7,46 |
| " | NaOH, 100° | 8 | 88,2 | 40,7 | 53,9 | 7,62 |
| " | NaOH, 100° | 20 | 79,9 | kein Rest | 100,0 | 7,95 |
| " | NaOH, 100° | 27 | 88,9 | 3,8 | 95,7 | 7,34 |
| Belebtschlamm | --- | 7 | 84,8 | 77,7 | 8,4 | 8,15 |
| " | --- | 20 | 108,8 | 68,6 | 36,9 | 6,59 |
| " | --- | 27 | 80,7 | 23,8 | 70,5 | 6,82 |

| Organismus | Vorbehandlung | Dauer [d] | Einwaage [mg] | Rückwaage [mg] | Abbau [%] | pH-Wert bei Ernte |
|---|---|---|---|---|---|---|
| Belebtschlamm | HCl, RT | 7 | 107,4 | 95,9 | 10,7 | 8,06 |
| " | HCl, RT | 20 | 84,1 | 26,8 | 68,1 | 6,75 |
| " | HCl, RT | 27 | 82,0 | 22,4 | 72,7 | 6,64 |
| " | HCl, 100° | 7 | 90,5 | 82,2 | 9,2 | 8,05 |
| " | HCl, 100° | 20 | 82,4 | 32,6 | 60,4 | 6,72 |
| " | HCl, 100° | 27 | 103,1 | 40,7 | 60,5 | 6,61 |
| " | NaOH, RT | 7 | 100,2 | 91,0 | 9,2 | 8,14 |
| " | NaOH, RT | 20 | 101,6 | 45,0 | 55,7 | 6,73 |
| " | NaOH, RT | 27 | 81,3 | 38,6 | 52,5 | 6,56 |
| " | NaOH, 100° | 7 | 94,5 | 85,8 | 9,2 | 8,07 |
| " | NaOH, 100° | 20 | 90,8 | 56,1 | 38,2 | 6,56 |
| " | NaOH, 100° | 27 | 86,2 | 45,4 | 47,3 | 6,59 |

Tabelle 2: Aerober Abbau von pulverisierten PHF durch den Mikroorganismus SK 37

| Substrat | Vorbehandlung | Dauer [d] | Einwaage [mg] | Rückwaage [mg] | Abbau [%] | pH-Wert bei Ernte |
|---|---|---|---|---|---|---|
| PHB-Pulver | --- | 4 | 62,0 | 25,1 | 59,5 | 7,19 |
| " | --- | 6 | 64,2 | 18,9 | 70,6 | 7,08 |
| " | --- | 4 | 124,7 | 67,7 | 45,7 | 6,98 |
| " | --- | 6 | 125,9 | 55,4 | 56,0 | 6,88 |
| " | --- | 4 | 247,0 | 161,6 | 34,6 | 6,81 |
| " | --- | 6 | 247,1 | 146,2 | 40,8 | 6,78 |

Beispiel 3:

Abbau unzerkleinerter Polymere (Granulatform) durch den Stamm SK 37

3.1 Die Polymere (Biopol D300G, D400G und D600G) wurden jeweils in einer Menge von 3 Gewichtsprozent in mineralischer Nährlösung (vgl. Beispiel 1) eingesetzt und der Abbau durch den Mikroorganismenstamm SK 37 nach 4, 7 und 11 Tagen gravimetrisch bestimmt.

Es wurden Abbauleistungen von 27 % (nach 4 Tagen) bis 61 % (nach 11 Tagen) erzielt. D300G wurde dabei zu 27 - 36 %, D600G zu 36-48 % und D400G zu 42 - 61 % abgebaut.

3.2 Das Polymer D400G wurde in einem Gewichtsanteil von 5 % in mineralischer Nährlösung eingesetzt und der Abbau durch SK 37 nach 4,7 und 11 Tagen gravimetrisch bestimmt. Der pH-Wert der Nährlösung war durch Zugabe von Tris auf 8,5 erhöht worden.

Trotz der erhöhten Polymer-Einwaage wurde eine nahezu identische Abbauleistung (57 % nach 11 Tagen) durch SK 37 erzielt. Offenbar wurde diese Verbesserung durch die Erhöhung des Anfangs-pH-Wertes bewirkt.

Beispiel 4:

Abbau von mechanisch zerkleinerten Polymer-Granula mit verschiedenen Medienzusammensetzungen

Das Polymer Biopol D400G wurde mit einer Kaffeemühle mechanisch zerkleinert. Um eine einheitliche Korngröße zu erreichen, wurde das erhaltene Polymer-Pulver durch ein Sieb mit definierter Maschenweite (710 $\mu$m) gegeben und die zurückgehaltenen Partikel verworfen.

Das Grundmedium bestand aus einer mineralischen Nährlösung (siehe Beispiel 1) 100 mmol/l Tris/HCl pH 8,5 sowie 2 % Polymer-Pulver.

Unter diesen Bedingungen wurde mit dem Mikroorganismus SK 37 nach 3,5 und 7 Tagen eine Abbauleistung von 48,53 bzw. 52 % erreicht. Eine deutliche Verbesserung der Abbauleistung wurde durch Verdoppelung der Konzentration der Mineralsalzmischung erreicht (42, 67 bzw. 76 % Abbau nach 3,5 bzw. 7 Tagen). Zur weiteren Optimierung des Mediums wurde eine Vielzahl an Mineralsalzmischungen getestet. Die beste Testmischung bestand aus der Grundmischung bei Verdoppelung bei Ammonium-, Eisen- und Kalzium-Ionenkonzentration. Unter diesen Bedingungen wurde eine Abbauleistung von 50, 73 bzw. 82 % nach 3,4 bzw. 7 Tagen und damit eine weitere Steigerung erreicht.

**Patentansprüche**

1. Verfahren zur Beseitigung von durch biologische und potentiell infektiöse Substanzen kontaminierten Abfällen, deren feste Komponenten im wesentlichen aus Kunststoffen bestehen, ausgewählt aus der Gruppe bestehend aus Polyestern, Polylactiden, Polylactonen, Polycarbonaten und Gemischen davon, **dadurch gekennzeichnet,**
daß man die Abfälle einer Behandlung unterzieht, die eine Inkubation in einem wäßrigen Medium in Gegenwart von Mikroorganismen oder/und ein Erhitzen in einem wäßrigen alkalischen Medium für eine ausreichende Zeitdauer umfaßt, um eine mindestens teilweise Auflösung der festen Kunststoffe zu bewirken.

2. Verfahren nach Anspruch 1,
**dadurch gekennzeichnet,**
daß nach der Behandlung eine Sterilisation des wäßrigen Mediums erfolgt.

3. Verfahren nach Anspruch 1 oder 2,
**dadurch gekennzeichnet,**
daß die Kunststoffe aus Polyhydroxyfettsäuren ausgewählt werden.

4. Verfahren nach einem der Ansprüche 1 bis 3,
**dadurch gekennzeichnet,**
daß die Behandlung eine Inkubation der Kunststoffabfälle in einem wäßrigen Medium in Gegenwart von Mikroorganismen umfaßt.

EP 0 679 412 A1

5. Verfahren nach Anspruch 4,
   **dadurch gekennzeichnet,**
   daß man Kunststoffe aus Polyhydroxybuttersäure-Copolymeren verwendet.

6. Verfahren nach Anspruch 4 oder 5,
   **dadurch gekennzeichnet,**
   daß man ein Polyhydroxybuttersäure-Polyhydroxyvaleriansäure-Copolymer verwendet.

7. Verfahren nach Anspruch 6,
   **dadurch gekennzeichnet,**
   daß man ein Polyhydroxybuttersäure-Polyhydroxyvaleriansäure-Copolymer mit einem Gewichtsanteil von 1 bis 50 % Hydroxyvaleriansäure verwendet.

8. Verfahren nach Anspruch 7,
   **dadurch gekennzeichnet,**
   daß man ein Polyhydroxybuttersäure-Polyhydroxyvaleriansäure-Copolymer mit einem Gewichtsanteil von 5 bis 30 % Hydroxyvaleriansäure verwendet.

9. Verfahren nach einem der Ansprüche 4 bis 8,
   **dadurch gekennzeichnet,**
   daß man den Kunststoff in einem Gewichtsanteil von 0,01 bis 5 % des Gewichts des wäßrigen Mediums einsetzt.

10. Verfahren nach Anspruch 9,
    **dadurch gekennzeichnet,**
    daß man den Kunststoff in einem Gewichtsanteil von 0,1 bis 1,5 % des Gewichts des wäßrigen Mediums einsetzt.

11. Verfahren nach einem der Ansprüche 4 bis 10,
    **dadurch gekennzeichnet,**
    daß die Behandlung bei einer Temperatur von 20 bis 40 ° C erfolgt.

12. Verfahren nach einem der Ansprüche 4 bis 11,
    **dadurch gekennzeichnet,**
    daß man die mikrobielle Behandlung mit einem Polyhydroxyfettsäuren-verwertenden Mikroorganismus durchführt.

13. Verfahren nach einem der vorhergehenden Ansprüche ,
    **dadurch gekennzeichnet,**
    daß man die mikrobielle Behandlung mit dem Isolat SK 37 (DSM 8962) oder einem äquivalenten Mikroorganismus durchführt.

14. Verfahren nach einem der vorhergehenden Ansprüche,
    **dadurch gekennzeichnet,**
    daß man die Kunststoffe als Formteile, Granulate oder/und als Pulver einsetzt.

15. Verfahren nach den Ansprüchen 4 bis 14,
    **dadurch gekennzeichnet,**
    daß eine Verringerung des Gewichts der festen Kunststoffe um mindestens 50 % innerhalb einer Behandlungsdauer von maximal 30 Tagen erfolgt.

16. Verfahren nach Anspruch 15,
    **dadurch gekennzeichnet,**
    daß eine Verringerung des Gewichts der festen Kunststoffe um mindestens 50 % innerhalb einer Behandlungsdauer von maximal 20 Tagen erfolgt.

17. Verfahren nach Anspruch 15,
    **dadurch gekennzeichnet,**

14

daß eine Verringerung des Gewichts der festen Kunststoffe um mindestens 50 % innerhalb einer Behandlungsdauer von maximal 10 Tagen erfolgt.

18. Verfahren nach einem der Ansprüche 15 bis 17,
**dadurch gekennzeichnet,**
daß das Gewicht der festen Kunststoffe um mindestens 80 % verringert wird.

19. Verfahren nach Anspruch 18,
**dadurch gekennzeichnet,**
daß die festen Kunststoffe völlig aufgelöst werden.

20. Verfahren nach einem der Ansprüche 4 bis 19,
**dadurch gekennzeichnet,**
daß man das Verfahren kontinuierlich unter einer Kontrolle des pH-Werts auf einen Bereich von 6 bis 9,5 durchführt.

21. Mikroorganismus SK 37 (DSM 8962)

22. Verwendung des Mikroorganismus SK 37 (DSM 8962) oder von Mikroorganismen mit äquivalenten Abbauleistungen zur mindestens teilweisen Verflüssigung von biologisch abbaubaren Kunststoffen in einem wäßrigen Medium.

23. Verfahren nach den Ansprüchen 1 bis 3,
**dadurch gekennzeichnet,**
daß die Behandlung ein Erhitzen in einem wäßrigen alkalischen Medium umfaßt.

24. Verfahren nach Anspruch 23,
**dadurch gekennzeichnet,**
daß man Kunststoffe verwendet, in denen feste anorganische Partikel dispergiert sind.

25. Verfahren nach Anspruch 24,
**dadurch gekennzeichnet,**
daß der Gewichtsanteil der anorganischen Partikel 0,1 bis 15 % auf Basis des Kunststoffgesamtgewichts beträgt.

26. Verfahren nach Anspruch 25,
**dadurch gekennzeichnet,**
daß der Gewichtsanteil der anorganischen Partikel 1 bis 10 Gew.-% beträgt.

27. Verfahren nach einem der Ansprüche 24 bis 26,
**dadurch gekennzeichnet,**
daß die Größe der anorganischen Partikel im Bereich von 1 nm bis 100 $\mu$m ist.

28. Verfahren nach einem der Ansprüche 24 bis 27,
**dadurch gekennzeichnet,**
daß die Behandlung ein Erhitzen auf mindestens 90 °C umfaßt.

29. Verfahren nach einem der Ansprüche 24 bis 28,
**dadurch gekennzeichnet,**
daß das wäßrige alkalische Medium einen pH-Wert von mindestens 13 aufweist.

30. Verfahren nach einem der Ansprüche 24 bis 29,
**dadurch gekennzeichnet,**
daß das wäßrige Medium mindestens 0,1 mol/l Alkalimetallhydroxid enthält.

31. Verfahren nach einem der Ansprüche 24 bis 30,
**dadurch gekennzeichnet,**
daß das Erhitzen für eine Dauer von 30 Minuten bis 24 Stunden erfolgt.

**32.** Verfahren nach einem der vorhergehenden Ansprüche,
**dadurch gekennzeichnet,**
daß die Behandlung der Kunststoffabfälle die Kombination einer Inkubation in Gegenwart von Mikroorganismen und eines Erhitzens unter alkalischen Bedingungen umfaßt.

**33.** Verwendung von Polyhydroxybuttersäure-Copolymeren zur Herstellung von Kunststoff-Labormaterialien, die innerhalb eines Zeitraums von 20 Tagen mikrobiell vollständig abbaubar sind.

**34.** Verwendung von Polyhydroxyfettsäure-Polymeren, die dispergierte anorganische Partikel enthalten, zur Herstellung von Kunststoff-Labormaterialien, die innerhalb eines Zeitraums von 24 Stunden durch Erhitzen unter alkalischen Bedingungen vollständig abbaubar sind.

**35.** Verwendung nach Anspruch 33 oder 34,
**dadurch gekennzeichnet,**
daß die Kunststoff-Labormaterialien Formteile sind, ausgewählt aus der Gruppe, umfassend Pipettenspitzen, Reaktionsgefäße, Küvetten und Mikrotiterplatten.

**Abb. 1:** Abbau von pulverisiertem Copolymer durch Isolat SK37

Der Abbau von Copolymer Pulver durch SK37 in Abhängigkeit von der Polymerkonzentration wurde nach 2 bzw. 4 Tagen gravimetrisch gemessen.

Europäisches
Patentamt

**EUROPÄISCHER RECHERCHENBERICHT**

Nummer der Anmeldung

EP 95 10 6382

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | CHEMICAL ABSTRACTS, vol. 118, no. 15, 12.April 1993 Columbus, Ohio, US; abstract no. 142267, JENDROSSEK, DIETER ET AL 'Degradation of poly(3-hydroxybutyrate), PHB, by bacteria and purification of a novel PHB depolymerase from Comamonas sp' * Zusammenfassung * & J. ENVIRON. POLYM. DEGRAD. (1993), 1(1), 53-63 CODEN: JEPDED, 1993 --- | 1-20,22, 32-35 | A62D3/00 |
| X | CHEMICAL ABSTRACTS, vol. 122, no. 16, 17.April 1995 Columbus, Ohio, US; abstract no. 189105, SCHERER, THOMAS M. ET AL 'Characterization and enzymic degradation of a crosslinked bacterial polyester' * Zusammenfassung * & J. ENVIRON. POLYM. DEGRAD. (1994), 2(4), 263-9 CODEN: JEPDED;ISSN: 1064-7564, 1994 --- | 1-20,22, 32-35 | |
| X | US-A-3 317 519 (S.D.LAZARUS ET AL.) * Ansprüche * ----- | 1-3, 23-31 | **RECHERCHIERTE SACHGEBIETE (Int.Cl.6)** A62D |

Der vorliegende Recherchenbericht wurde für alle Patentansprüche erstellt

| Recherchenort | Abschlußdatum der Recherche | Prüfer |
|---|---|---|
| DEN HAAG | 27.Juni 1995 | Dalkafouki, A |